# EUROPEAN PATENT SPECIFICATION

(11) **EP 2 753 684 B1**
(45) Date of publication and mention of the grant of the patent: **31.07.2019**
(21) Application number: 12829917.9
(22) Date of filing: 10.09.2012
(51) Int. Cl.: C12M 1/34

(54) **A MULTILEVEL ANALYTE ASSAY**
MEHRSTUFIGES ANALYT-ASSAY
DOSAGE DE SUBSTANCE À ANALYSER À PLUSIEURS NIVEAUX

(30) Priority: 08.09.2011 US 201161532433 P
(43) Date of publication of application: 16.07.2014
(73) Proprietor: Nexus Dx, Inc., San Diego, CA 92121 (US)
(72) Inventor: BRENNAN, Ed, San Diego, California 92121 (US); LI, Conan, San Diego, California 92121 (US); BELENKY, Alexander, San Diego, California 92121 (US)
(74) Representative: CMS Cameron McKenna Nabarro Olswang LLP
(86) International application number: PCT/US2012/054425
(87) International publication number: WO 2013/036913

(56) References cited:
- EP-A1- 0 060 700
- EP-A1- 0 465 266
- EP-A1- 1 197 754
- EP-A2- 0 553 770
- EP-A2- 2 031 376
- WO-A1-00/42434
- WO-A1-98/36278
- WO-A1-2004/086042
- WO-A1-2004/095030
- WO-A1-2006/065314
- WO-A1-2008/122796
- WO-A1-2009/144507
- WO-A1-2011/100462
- US-A- 5 968 839
- US-A1- 2004 029 177
- US-A1- 2006 019 404
- US-B1- 6 656 745
- NAOYA OHMURA ET AL: "Combinational use of antibody affinities in an immunoassay for extension of dynamic range and detection of multiple analytes", ANALYTICAL CHEMISTRY, AMERICAN CHEMICAL SOCIETY, vol. 75, no. 1, 1 January 2003 (2003-01-01), pages 104-110, XP002631118, ISSN: 0003-2700, DOI: 10.1021/AC020247 [retrieved on 2002-11-23]
- DI ANGELANTONIO ET AL.: 'Prognostic significance of admission levels of troponin I in patients with acute ischaemic stroke' J NEUROL NEUROSURG PSYCHIATRY vol. 76, 2005, pages 76 - 81, XP055144334
- GANTELIUS J ET AL: "A lateral flow protein microarray for rapid determination of contagious bovine pleuropneumonia status in bovine serum", JOURNAL OF MICROBIOLOGICAL METHODS, ELSEVIER, AMSTERDAM, NL, vol. 82, no. 1, 1 July 2010 (2010-07-01), pages 11-18, XP027265479, ISSN: 0167-7012 [retrieved on 2010-07-01]

## Description

### BACKGROUND OF THE INVENTION

Tests have been developed to detect the presence of analytes in bodily samples, including in point-of-care formats that allow health care professions to quickly determine a patient's condition. These tests typically involve antigen-antibody reactions or some other ligand-ligand receptor binding event, and may involve radioactive, enzymatic, fluorescent, or other detectable labels. Many such tests are designed to make a qualitative determination by detecting the presence or absence of the analyte in the sample at or above a specified level or concentration.

However, a binary test result indicating that an analyte is present or absent in a sample at or above a detectable concentration does not necessarily provide the most useful information to the clinician, the patient, or the person administering the test. For example, the presence of cardiac troponins (e.g., cTnI), which are markers of myocardial damage, in patient serum can be indicative of, or associated with, numerous conditions, including myocardial infarction as well as secondary ischemic and non-ischemic injuries such as coronary intervention, myopericarditis, septicemia, cardiac trauma, chemotherapy, and renal failure. See Sharma et al., Cardiac Troponins, J. Clin. Pathol. 57:1025-1026 (2004). Thus, the presence of cardiac troponins in serum does not necessarily indicate that a patient is experiencing an acute cardiac event (e.g., myocardial infarction or MI). Further, the absence of such levels does not necessarily exclude an acute cardiac event (such as MI), since troponin levels increase in serum over the course of about 4 to 12 hours after such an event. It is instead important to determine whether troponin levels are rising over time in a manner consistent with an acute cardiac event.

WO00/42434 describes a test strip having at least two detection areas, at least the first area having a predetermined capacity for binding analyte. When the capacity of the first area is exceeded, the analyte is bound by a second area of at least equal capacity. WO 2004/086042 describes multichannel radial lateral flow devices for marker proteins.

A point-of-care test that has the ability to provide accurate, semi-quantitative, convenient, and cost effective information concerning the level of analytes in samples, including markers of myocardial injury, would fill a great need.

### SUMMARY OF THE INVENTION

The present invention provides methods and devices for detecting the presence of two or more threshold levels of an analyte in a sample. In various examples, there is provided convenient point-of-care tests for determining the condition of a patient, so as to guide cost effective health care.

In one aspect, the present invention provides a device for the detection of at least two threshold levels of a marker of myocardial damage or injury in a patient sample. Rising levels of such markers of myocardial injury or damage, which include cardiac troponins (e.g., cTnT or cTnI), myoglobin and CK-MB, can be indicative of myocardial infarction and other acute cardiac events described herein. The device may be suitable for a point-of-care determination, and may be administered by a health care professional or by an untrained person, to determine the need for hospitalization or emergency care.

In accordance with this aspect, the device involves a lateral flow membrane and binding reagents to form a detectable complex in the presence of a marker of myocardial injury or damage ("biomarker"). The device comprises a sample application zone, and one or more fluid flow paths in fluid communication with the sample application zone. The fluid flow paths comprise a first biomarker capture zone and a second biomarker capture zone, which may be in series on a single flow path or may be in separate flow paths. The first capture zone is configured to detectably capture the biomarker when present in the sample at or above a first threshold level, and the second capture zone is configured to detectably capture the biomarker when present in the sample at or above a second threshold level. Thus, the device provides semi-quantitative information for determining or tracking the biomarker levels in a patient, and provides versatile information for determining the condition of the patient in a point-of-care format.

The device contains binding reagents for the detection of at least two threshold levels of a cardiac troponin, such as cTnI. The first threshold level is indicative of the presence of a cardiac troponin in blood circulation, and corresponds to about 0.04 ng/mL and provide for more specific clinical evaluation. For example, a level of cTnI above this threshold indicates that the patient should be monitored for rising cTnI levels, which is indicative of a potential cardiac event. The second threshold level is indicative of an acute cardiac event, and corresponds to about 0.1 ng/mL of a cardiac troponin in the circulation. A level of cTnI above the second threshold level is indicative of an acute cardiac event, such as MI. The device may also provide for a determination of three or more threshold levels of cTnI in certain examples, to enhance the sensitivity and/or specificity and/or range of the test, and/or to provide for an even further specific clinical evaluation.

The fluid flow path(s) are formed by one or more bibulous, absorbent, and/or porous membranes, and each flow path may include a control zone that produces a detectable signal as an indication of successful sample flow. The fluid flow path further includes membranes or pads of various porosity to filter cells and/or debris, which are located in proximity to the one sample application zone. In these or other examples, the fluid flow path may comprise one or more reagent pads housing binding reagents, and/or may include an absorbent pad at the terminal end to help drive sample flow. The one or more fluid flow paths comprise a first marker capture zone and a second marker capture zone, wherein the first and second capture zones are in series on a single flow path or are in separate flow paths. The first capture zone is configured to detectably capture the marker when present in the sample at or above a first threshold level, and the second capture zone is configured to detectably capture the marker when present in the sample at or above a second threshold.

The biomarker (e.g., cTnI) is detectable in the sample by formation of a capturable and detectable complex, formed with a first binding member and a second binding member that are included in the device (e.g., in the reagent pad) or added to the sample (e.g., in a preincubation step). The first binding member and the second binding member can be immunoreagents as described in detail herein. The first binding member has an attached or bound detectable label which can be visualized or detected upon immobilization of the complex at a capture zone. Various suitable labels are described herein. The device may include one or a plurality of second binding members to allow capture or immobilization of the complex at the specific capture zones. Particularly, each second binding member is either immobilized as a continuous area formed by deposited second binding member along the capture zones or is capable of being immobilized during sample flow as a continuous area formed by deposited second binding member along the capture zones. The marker is detectable in the sample by formation of a capturable and detectable complex formed with the first binding member and the second binding member that are included in the device. For example, at least one second binding member may be capturable at a capture zone by a streptavidin-biotin interaction, an antibody-antigen interaction, or by hybridization of complementary or substantially complementary oligonucleotides. The first and/or second binding members may be diffusively bound in the flow path(s) upstream of the cTnI capture zone(s), such that the biomarker in the sample forms capturable and detectable complexes with the first and second binding members during sample flow.

The first capture zone and the second capture zone are in different, parallel or bidirectional, flow paths in fluid communication with the sample application zone. For example, the device may comprise two, three, or more flow paths, each configured to determine a different threshold level of the biomarker in the sample. In some examples, the device may comprise at least a second sample application zone, and corresponding flow path or flow paths, for repeating the test within a period of time to detect rising analyte levels.

The device may be configured for the detection of the biomarker at a plurality of threshold levels. In some examples, the first capture zone has a first amount of immobilized capture agent, and the second capture zone has a second amount of said immobilized capture agent, and so on for additional capture zones, so as to detectably capture different threshold levels of the marker at each capture zone. The immobilized capture agent may be, for example, streptavidin, biotin, an antibody, or a natural or artificial polynucleotide, with the opposing binding member being conjugated to an analyte-specific binding reagent ("the second binding member"). In other examples, the first capture zone comprises an antibody with a first affinity for the biomarker, and the second capture zone comprises an antibody with a second affinity for the biomarker, so as to detectably capture the biomarker at different threshold levels. Low and high affinity antibodies may be non-diffusively bound to respective capture zones, or may be directed to particular capture zones through the use of specific capture agents, such as streptavidin or polynucleotide capture agent(s), among others. For example, polynucleotide capture agents having different sequences may be used to direct low and/or high affinity antibodies to particular capture zones.

The capture zones are continuous, with threshold or relative levels being identified by the relative length of the detectable immunocomplex captured along the flow path.

In another aspect, the invention provides a semi-quantitative method for evaluating the level of a marker of myocardial injury or damage in a patient sample, such as a cardiac troponin (e.g., cTnI). The method comprises applying a test sample to a device of the present invention, and detecting the presence or absence of a specific detectable signal in the continuous capture zones, and determining a plurality of threshold levels of the marker, wherein the plurality of thresholds comprises a first threshold level and a second threshold level of the marker. The patient may have one or more symptoms of an acute cardiac event, and/or may have a chronic heart condition, making a rapid troponin (e.g., cTnI) determination potentially critical for determining the need for emergency intervention. Further, the patient may have a chronic medical condition associated with specific circulating levels of cTnI, such as renal disease, previous cardiac trauma or surgical intervention, or septicemia, such that a method for quickly and accurately detecting rising levels of cTnI over potential background levels would be desirable to avoid the expense of unnecessary emergency care. Where the patient sample shows a level of the marker at the first threshold level, but below the second threshold level, the test is repeated within about one to about ten hours to detect rising levels of the marker.

For example, a detectable signal indicating a first threshold level of cTnI is suggestive that the patient be monitored for signs and symptoms of a myocardial infarction, and in some examples, the test is repeated after a period of time to detect rising levels of cTnI. A detectable signal indicating a second threshold level of cTnI is suggestive that the patient be treated for a potential acute cardiac event, such as an MI. Where the patient does not have a level of cTnI detectable with the test device, the patient is determined to not be experiencing an acute cardiac event, or has a low risk of an adverse outcome.

In another aspect, there is provided a device and method for the detection of at least two threshold levels of an analyte in a test sample. In this aspect, the analyte need not be a marker of myocardial injury, but may be any marker whose level in a sample can suggest more than one medical treatment decision point. Such markers may be indicative of stroke or brain trauma, an allergic response to a therapeutic agent (e.g., IgE antibodies to a therapeutic agent), or a measure of a particular disease activity, including autoimmune disease by detection of autoantibodies or determination of cytokine profiles.

In this aspect, the device generally comprises a sample application zone, and one or more fluid flow paths in fluid communication with the sample application zone. The fluid flow path(s) include a first analyte capture zone and a second analyte capture zone. The first and second capture zones are in series on a single flow path or are in separate flow paths. The first analyte capture zone comprises a first amount of an immobilized capture agent to detectably capture, directly or indirectly, the analyte (e.g., as present in a detectable complex) when present in the sample at or above a first threshold level. The second analyte capture zone comprises a second amount of the immobilized capture agent configured to detectably capture, directly or indirectly, the analyte (e.g., as present in a detectable complex) when present in the sample at or above a second threshold level. Binding reagents for forming detectable complexes (e.g., immunocomplexes) with the analyte may be present in the device in the fluid flow path, or combined with the sample in a preincubation step.

In various examples, the immobilized capture agent is one member of a binding pair selected from streptavidin-biotin, antibody-antigen, or complementary or substantially complementary natural or artificial oligonucleotides, with the opposing binding member being conjugated to one of the binding reagents for forming the complex (e.g., the second binding member).

### BRIEF DESCRIPTION OF THE FIGURES

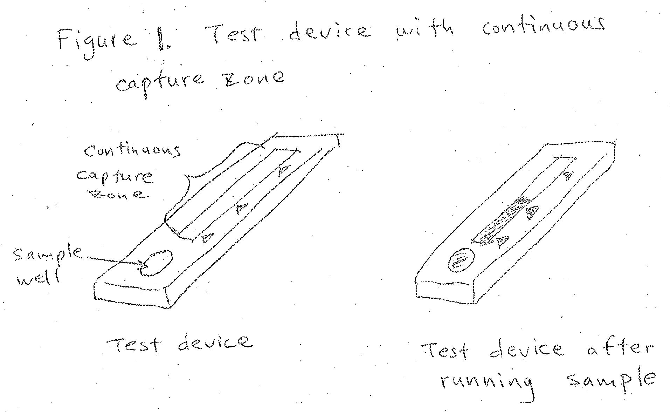

**Figure 1** illustrates a test device of the present invention. The test device includes a sample application zone in fluid communication with a lateral flow membrane. Figure 1 further illustrates an embodiment using a continuous capture zone.

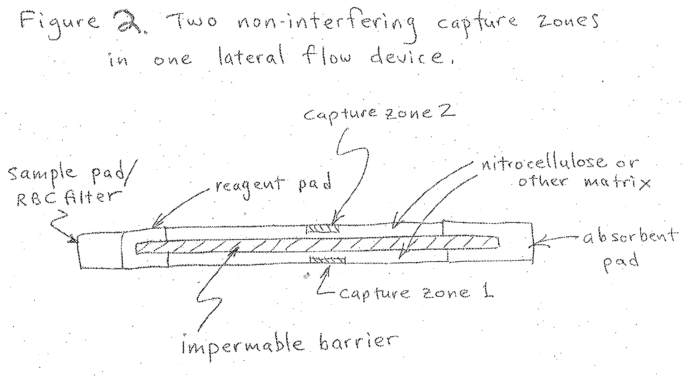

**Figure 2** illustrates a test device of the present invention. The test device includes a sample application zone in fluid communication with one lateral flow membrane, separated into two subsections by an impermeable barrier.

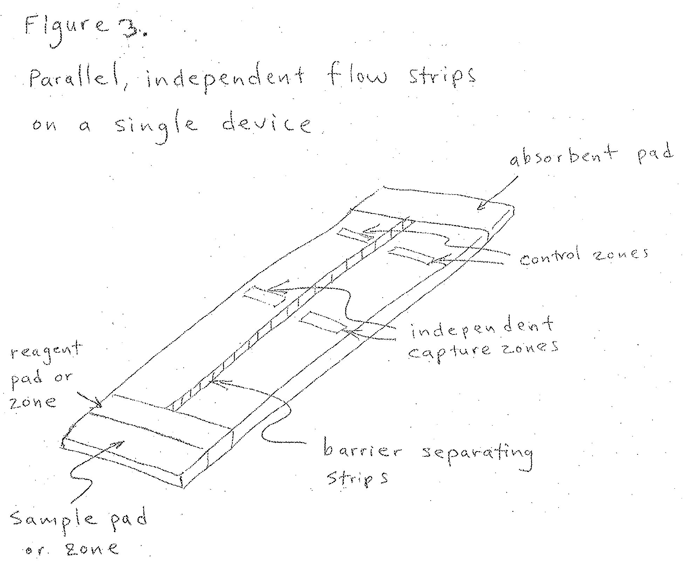

**Figure 3** illustrates a test device of the present invention. The test device includes a sample application zone and a reagent pad, the latter of which is in fluid communication with two parallel, independent lateral flow membranes, separated by a barrier. Each lateral flow membrane includes a capture zone and a control zone, and downstream of each lateral flow membrane is an absorbent pad.

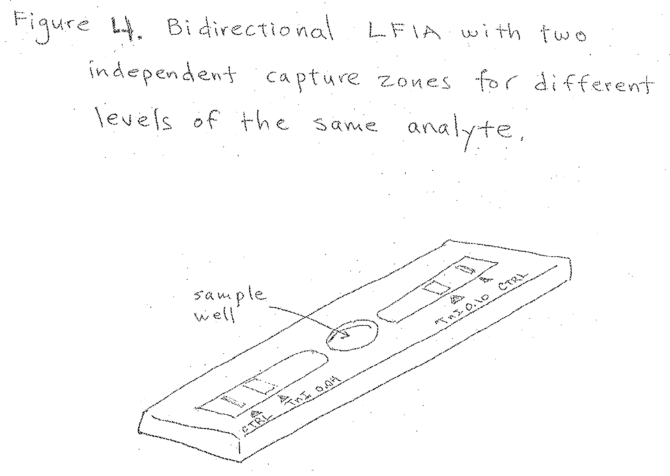

**Figure 4** illustrates a test device of the present invention. The test device includes two independent lateral flow membranes in fluid communication with a single sample application zone. The test device employs bidirectional flow.

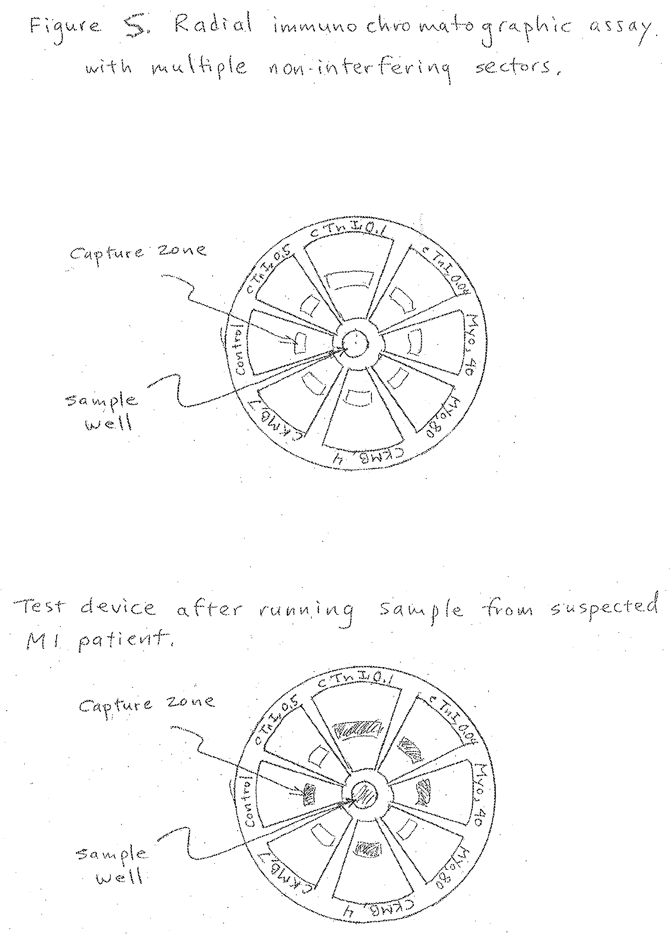

**Figure 5** illustrates a test device of the present invention. The test device includes one sample application zone and a plurality of lateral flow membranes arranged radially around the sample application zone for detecting a plurality of analytes, each analyte at a plurality of threshold levels.

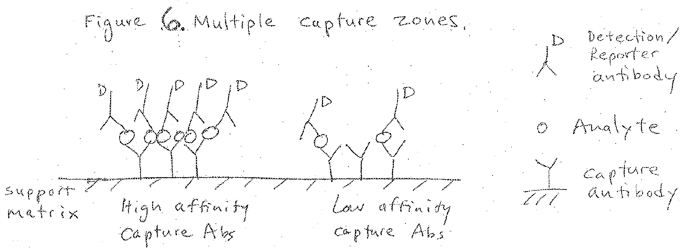

**Figure 6** illustrates a test device comprising a lateral flow membrane comprising two serially oriented capture zones, supported by a support matrix. The two capture zones employ high and low affinity antibodies to detect the low and high analyte thresholds, respectively. The test device is designed to detectably capture two different concentrations of the same analyte.

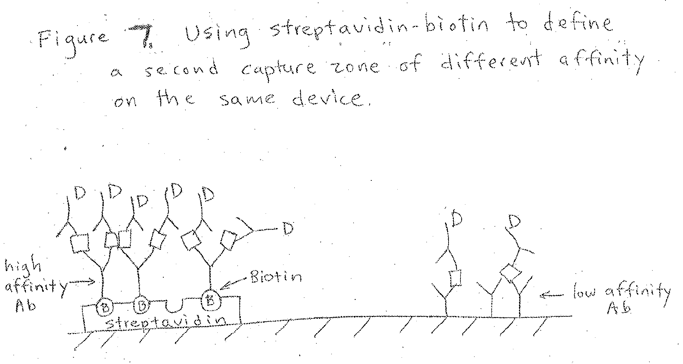

**Figure 7** illustrates a lateral flow membrane comprising two capture zones, designed to detectably capture two different concentrations of the same analyte. One capture zone employs high affinity antibodies, each conjugated to biotin. The biotin is bound to streptavidin on the lateral flow membrane, to immobilize the high affinity antibodies. The second capture zone employs low affinity antibodies bound directly to the lateral flow membrane.

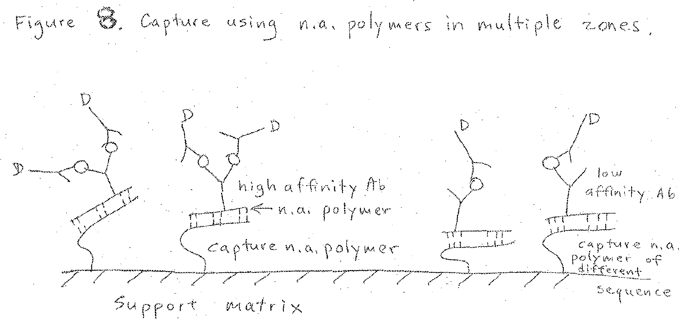

**Figure 8** illustrates a lateral flow membrane comprising two capture zones, designed to detectably capture two different concentrations of the same analyte, or two different analytes. The capture agents are oligonucleotides, where the complementary oligonucleotides are immobilized at particular capture zones.

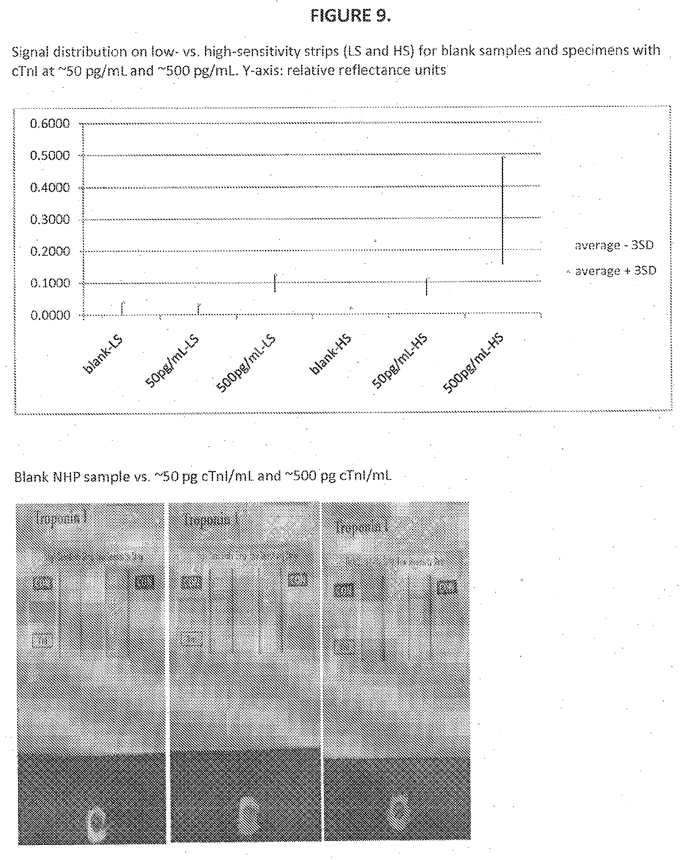

**Figure 9** shows the results from a multi-level cardiac troponin assay run on a test device of the present invention. The exemplary embodiment employs parallel flow paths and different amounts of biotin immobilized at the capture zones to create two detectable thresholds of analyte.

### DETAILED DESCRIPTION OF THE INVENTION

The present invention provides methods and devices for detecting the presence of two or more threshold levels of an analyte in a sample. In various examples, the invention provides convenient point-of-care tests for determining the condition of a patient, so as to guide cost effective health care.

Analyte concentrations in bodily fluid samples are useful indicators for diagnosis or prognosis of disease and other clinical conditions. As described herein, multiple threshold (*i.e.,* cutoff) levels on a single test device enhances the information delivered by the test, and provides the opportunity to quickly evaluate medical treatment options. Furthermore, measurement of an analyte or analytes at multiple threshold levels may be useful to monitor progression of a disease state or medical condition, or to determine the presence or absence of a medical condition, by measuring the evolution of the analyte level through serial measurements over a period of time.

Measurement of an analyte concentration against multiple threshold levels using a semi-quantitative test, such as a lateral flow test, may be particularly useful, since more than one clinical decision point (and therefore, the resultant course of action) can be addressed in a single evaluation at the point of care, and may be conducted by an untrained person.

In various embodiments, the device and methods of the invention avoid the requirement for cumbersome or inconvenient instrumentation often required for quantitative analysis, making the invention ideal for the home, rural areas, ambulances, airplanes, and underdeveloped locales, for example. The invention provides an easily interpretable test sufficient to signal to an inexperienced user the need for emergency health care.

### Analytes and Markers of Interest

The device detects analytes associated with myocardial injury or damage, whose presence in serum are indicative of acute myocardial events, such as myocardial infarction. Such analytes include cardiac troponins (e.g., cTnT, cTnI, and cTnC), myoglobin, and CK-MB. Other markers of myocardial injury are described in U.S. Patent 6,461,828. The device may be configured to detect a plurality of threshold levels of at least one, two, or three markers of myocardial injury.

In one embodiment, the analyte is a cardiac troponin (e.g., cTnI, cTnC, cTnT). The level of circulating cardiac troponins is specific for myocardial damage. Further, the level of the cardiac troponins in serum changes over time in the progressive development of MI. For example, if a patient presents with MI-like symptoms such as unstable chest pain and non-specific changes on ECG but has a blood concentration of cTnI lower than 99^{th} percentile of normal when symptoms first appear, then the subject should be tested again in six to nine hours. An increase in cTnI over time provides more accurate risk assessment and specificity for myocardial necrosis, and rules out non-specific causes of elevated troponin such as renal damage. An increase in cTnI of 20% to 30% on successive tests is considered significant.

The device determines the presence of a cardiac troponin, such as cTnI, in a patient sample above at least two threshold levels. Cardiac troponin I (cTnI) increases for the first 4 to 12 hours after a myocardial infarction (MI). For example, if a subject's circulating troponin I level is greater than about 0.04 ng/mL but is less than about 0.1 ng/mL, there is a suspicion of myocardial infarction or acute cardiac event, but such a level of troponin I could be due to causes other than myocardial infarction such as renal disease. Thus, a single measurement reporting a blood cTnI amount that is greater than 0.04 ng/mL, but less than 0.1 ng/mL, may not be definitive. If, however, several hours after the first measurement (e.g., from two to eight hours later), a second measurement shows that the subject's cTnI level is greater than about 0.1 ng/mL, this indicates that the subject's troponin I level has increased over time, and that an MI is significantly more likely.

Therefore, in particular examples related to cTnI, the device is configured to detect a low threshold level of cTnI in patient blood or serum, the low threshold level being indicative of the presence of cTnI. This threshold is about 0.04 ng/mL and may be from about 0.01 ng/mL to about 0.06 ng/mL. In these embodiments, the device is also configured to simultaneously detect a higher threshold of cTnI that is indicative of an acute cardiac event, such as MI. The higher threshold of cTnI is about 0.1 ng/mL or may correlate to about 0.08 ng/mL to about 0.2 ng/mL in patient blood or serum . If the initial measurement with the test device shows cTnI levels greater than about 0.1 ng/mL, then the risk of an adverse event such as MI or death is about 2.2- to 3.0-fold higher as compared to if cTnI were less than 0.1 ng/mL. If the initial measurement shows cTnI levels in blood or serum are less than about 0.04 ng/mL, then an MI would be highly unlikely, unless the test was performed very early in the development of MI.

In some examples, the device is configured to determine the presence of a third threshold level of circulating cTnI. The third threshold level may be from about 0.2 ng/mL to about 0.7 ng/mL (e.g., about 0.5 ng/mL). A level of cTnI of approximately 0.5 ng/mL or more has a high specificity for MI. A subject whose blood or serum cTnI levels is about 0.5 ng/mL or more would likely be experiencing myocardial damage. This system of three threshold levels may be particularly meaningful for the individual performing the test at home following the onset of acute chest pain or other symptoms of MI, including for patients that have a history of acute and/or chronic heart conditions, and/or who experience other ailments for which cTnI may be present in the circulation, such as renal disease or prior cardiac trauma or intervention, or carditis (pericarditis or endocarditis). In the absence of other symptoms or indications, the test result may provide information regarding the need to seek immediate medical care.

In some examples, at least two analytes are detected by a single test device. The two analytes may be specific markers for MI. For example, the test device comprises at least one capture zone, specific for troponin I, and at least a second capture zone, specific for troponin T. The detectable thresholds for each troponin are such that one is selected to "rule in" MI (a low threshold indicating the presence of a circulating troponin), while the other is selected to "rule out" MI (a high threshold that is indicative of MI or an acute cardiac event). Since both troponin I and troponin T are specific for MI, but are genetically coded for by different genes, depletion of one molecule by its specific capture zone on a lateral flow device would not affect the levels of the other molecule for detection by its specific capture zone on that device.

The device may be configured to determine at least one, or a plurality of threshold levels for a second marker of myocardial injury or status, such as myoglobin or CK-MB. Such markers are described in U.S. Patent 5,290,678.

In these and other examples, the device can provide for a semi-quantitative determination of an additional marker of cardiac status, such as NT-proBNP, BNP, proBNP, ANP, or pro-ANP, as also described in U.S. Patent 6,461,828.

In other examples, the device determines a plurality of threshold levels for one or more markers of brain injury or trauma, as a determination of stroke or other brain trauma. For example, the markers include one or more of myelin basic protein (MBP), the beta isoform of S 100 protein (S 100), neuronal specific enolase (NSE), Thrombomodulin, Glucose Transporter I in the dimeric or tetrameric form, Neurothelin, Gamma Glutamyl Transpeptidase, and P-glycoprotein. These and other markers are described in U.S. Patent 6,780,606. Thus, described are devices and methods for tracking the circulating levels over time, as described and exemplified herein for cardiac markers.

In still other examples, the device determines the presence of inflammatory or immune response markers, which may be monitored upon exposure to a drug or potential antigen, or other stimulus. Such markers may be antibodies against a therapeutic agent to monitor efficacy of, or reaction to, a therapeutic intervention, or the marker may be an autoantibody indicative of an autoimmune disease to monitor disease activity over time, including in response to therapy.

Generally, the analyte may be a toxin, an organic compound, a protein (including an immunoglobulin), peptide (e.g., peptide hormone), microorganism or component thereof, vitamin, metabolite, drug (including those administered for therapeutic purposes as well as those taken for illicit purposes), pollutant, pesticide, or antibodies to any of the foregoing.

Generally, the sample may be any material to be tested for the presence and/or concentration of an analyte, including a biological or environmental sample. In various examples, the sample is a body fluid sample such as whole blood, serum, plasma, urine, sweat, saliva, cerebrospinal fluid, nasal secretions, or the like. Where the device is configured to detect one or more markers of myocardial injury, the sample may be blood, serum, or plasma.

### Lateral Flow Tests

The device and methods of the invention employ a lateral flow test. Lateral flow tests are well known, and are described for example in U.S. Patents 6,461,828, 6,177,281, 7,390,674, and U.S. 7,491,551. The lateral flow test generally comprises a sample application zone (e.g., for applying fluid sample for testing), and one or more fluid flow paths in fluid communication with the sample application zone. The fluid flow paths comprise a first biomarker capture zone and a second biomarker capture zone, which are in series on a single flow path or are in separate flow paths. The term "fluid communication" means that the sample can flow from a first component to a second component of the flow path, or vice versa. A first component is in fluid communication with a second component, even if there is an intervening component. For example, a sample application zone, a reagent zone (e.g., that houses the assay immunoreagents), and lateral flow membrane that includes one or more capture zones, may be situated serially on a test device, and will be in fluid communication. The device further comprises one or more components for the separation of blood cells or large debris, including filters of appropriate porosity.

The lateral flow test involves a lateral flow membrane and binding reagents to form a detectable complex in the presence of the marker or analyte of interest. The complex (e.g., immunocomplex) comprises a first reagent specific for the analyte, and having an attached or bound label, and the second reagent is immobilized or capturable, and is also specific for the analyte of interest. The binding reagent may be an immunoreagent, which is generally an antibody or portion thereof. In various embodiments, the immunoreagent is a monoclonal antibody, a polyclonal antibody, multispecific or bispecific antibody, chimeric antibody, or antibody portion or fragment that binds to the analyte of interest. The antibody may be of any isotype, including IgA, IgD, IgE, IgG, and IgM, and subtype thereof.

The immunoreagent may further be an antigen-binding antibody portion or fragment, such as a Fab, Fab', F(ab')2, Fv fragment, a single-chain antibody molecule, a multispecific antibody formed from any fragment of an immunoglobulin molecule comprising one or more antigen-binding domains.

In embodiments where the binding reagents are immunoreagents, at least one immunoreagent (e.g., "the first immunoreagent" or "the first binding member") contains an attached or bound label detectable by visual or instrumental means. One or more labels can be used in the devices and methods described herein, e.g., in order to visually distinguish capture zones in different flow paths, or to distinguish control zones. In other examples, two distinct labels are used to visually differentiate different analytes. Various labels suitable for use in the present invention include labels which produce signals through either chemical or physical means. Such labels include enzymatic labels (e.g., that produce a detectable signal in the presence of substrate), chromogens, dyes, catalysts, fluorescent or fluorescent-like compounds and/or particles, magnetic compounds and/or particles, chemiluminescent compounds and or particles, and radioactive labels. The label may be covalently or non-covalently bound, directly or indirectly, to the first immunoreagent, which is specific for the target analyte. In certain examples, the label remains detectable for at least 2, at least 4, at least 6, or at least 8 hours after conducting the test, such that a repeat test several hours later can be directly compared to the first result.

At least one immunoreagent (e.g., "the second immunoreagent" or "second binding member") is immobilized at a capture zone, or is capturable at said capture zone through a specific binding pair interaction. Exemplary binding pair members include complementary or substantially complementary oligonucleotides, ligand-receptor, antibody-antigen, or streptavidin-biotin. This second binding member may provide the basis for detecting a plurality of threshold levels of the analyte. For example, as discussed in greater detail below, the capture zones may employ different amounts or concentrations of the same binding pair member so as to immobilize or capture the second immunoreagent at different levels. Alternatively, the capture zones may employ different immunoreagent capture schemes, so as to distinguish between second immunoreagents having different affinities for the analyte of interest. Particularly, low affinity and high affinity antibodies can be recognized by the identity of the conjugated capture reagent.

Each flow path may further include one or more control zones downstream of the one or more capture/detection zones as an indication of successful sample flow. The control zone may have a means for capturing or detecting the presence of unbound labeled binding reagent at the terminal end of the flow path. For example, the control zone may comprise immobilized anti-mouse IgG, where the first binding reagent (e.g., the labeled immunoreagent) is a mouse monoclonal antibody. Other sample flow controls are known in the art, and may be used in accordance with the invention.

The test device may further comprise an absorbent pad downstream of, and in fluid communication with, the lateral flow membrane to help drive sample flow. Such components for lateral flow assays are also well known.

Further still, additional components may be employed to premix sample and binding agent prior to application to the device. Exemplary components for this purpose are described in WO 2007/098184, WO 2009/014787, and WO 2010/132453.

### Membrane Substrates

The lateral flow substrates are generally porous, absorbent, and/or bibulous substrates or membranes that allow transport of fluid samples, e.g., by capillary action. A variety of materials can be used as the lateral flow substrate, and such materials include organic or inorganic polymers, and natural and synthetic polymers, including but not limited to, agarose, cellulose, nitrocellulose, cellulose acetate, other cellulose derivatives, dextran, dextran-derivatives and dextran co-polymers, other polysaccharides, glass, silica gels, gelatin, polyvinyl pyrrolidone (PVP), rayon, nylon, polyethylene, polypropylene, polybutylene, polycarbonate, polyesters, polyamides, vinyl polymers, polyvinylalcohols, polystyrene and polystyrene copolymers, polystyrene cross-linked with divinylbenzene or the like, acrylic resins, acrylates and acrylic acids, acrylamides, polyacrylamide, polyacrylamide blends, co-polymers of vinyl and acrylamide, methacrylates, methacrylate derivatives and co-polymers thereof.

In one example, the lateral flow substrate comprises nitrocellulose, e.g., any nitric acid ester of cellulose. In certain examples, the lateral flow substrate comprises nitrocellulose in combination with carboxylic acid esters of cellulose.

The pore size of the substrate (e.g., nitrocellulose substrate) may vary. For example, the pore size may be from about 1 µm to about 20 µm, or from about 8 µm to about 15 µm, or from about 10 µm to about 12 µm.

Various components of the flow path may employ different substrate materials, and/or different pore sizes to control the fluid flow, control the ability of reagents to diffusively bind to the substrate material, and/or control the filtration of debris.

Components of the flow path may be treated with blocking agents such as bovine serum albumin, whole animal serum, casein, or non-fat dry milk.

The lateral flow substrate defining the at least one fluid flow path may be backed by, or laminated upon, a generally water impervious layer, e.g., Mylar®. However, in some examples the lateral flow substrate may be self-supporting. When employed, the backing is generally fastened to the matrix by an adhesive. A water impervious backing is generally used for substrates of low thickness.

### Fluid Flow Paths

Generally, the fluid flow paths are defined by at least one sample application zone, and at least one lateral flow substrate. Where multiple lateral flow substrates are employed, the multiple lateral flow substrates may be in fluid communication with the same or different sample application zones. Reagent zones or pads are optional.

In one embodiment, the test device includes one lateral flow membrane in fluid communication with a single sample application zone **(****Figure 1**). However, the invention is not limited to such an architecture. For example, in another embodiment, the test device includes one lateral flow membrane as shown in **Figure 2****.** In this embodiment, the lateral flow membrane allows for two fluid flow paths, because an impermeable barrier divides the membrane into two parallel subsections.

In another embodiment, the test device includes two lateral flow membranes, as shown in **Figure 3****,** each defining a separate fluid flow path. In this embodiment, two independent flow strips are in parallel, on the same test device. Each lateral flow membrane is in fluid communication with a reagent pad (e.g., housing the immunoreagents), which is downstream of, and in fluid communication with, a single sample application zone. In one embodiment, the test device architecture is similar to that of **Figure 3**, except that no reagent zone is present, and each lateral flow membrane is in direct fluid communication with the sample application zone.

At least two separate sample application zones may also be employed, each in fluid communication with a separate, independent, lateral flow substrate, where each lateral flow substrate defines a separate fluid flow path. The separate fluid flow paths may further include separate reagent zones or pads. The test device may have additional sample application zones (e.g., three, four or five sample application zones), each corresponding to separate fluid flow paths on separate lateral flow substrates, or corresponding to separate fluid flow paths on the same lateral flow substrate (e.g., a lateral flow membrane divided into a plurality of subsections by one or more impermeable barriers). During use, a measured volume of fluid sample is applied to each sample application zone.

Parallel, independent lateral flow substrates may also be employed in a single test device by positioning a sample application zone as shown in **Figure 4**. In this embodiment, the test device employs bidirectional fluid flow. Similarly, multiple lateral flow membranes may be arranged radially around a single sample application zone (**Figure 5**). In this embodiment, each lateral flow membrane defines one fluid flow path, or defines multiple fluid flow paths through the use of one or more impermeable barriers.

In another embodiment, the test device includes one sample application zone for multiple lateral flow substrates. In this embodiment, the sample is applied to the test device once, and the sample application zone is in fluid communication with each of the lateral flow substrates or fluid flow paths (**Figures 3****,** **4****,** **5**).

In certain examples, the device has a design that enables two or three tests, such that tests may be conducted over the course of from 2 to 10 hours, to monitor increasing analyte concentrations.

### Capture Zones

The device and methods described herein employ at least two capture zones for discriminating two threshold levels of analyte. The at least two capture zones may be arranged serially on a single flow path, or may be in separate flow paths, each designed to capture a different detectable threshold concentration of the analyte.

One multiple capture zone embodiment is presented in **Figure 6. Figure 6** shows a side view of a fluid flow path on a lateral flow membrane with a support matrix. The lateral flow membrane comprises two serially oriented capture zones, where one capture zone employs a high affinity capture ligand (e.g. antibody), while the second capture zone employs a different low affinity capture ligand (e.g., antibody). The difference in affinity allows for the capture of different amounts of the same analyte on the same test device, thereby detecting the analyte at different threshold levels. As shown in Figure 6, the antibodies having different affinities (which correspond to the second immunoreagent), may be immobilized at respective capture zones. The embodiment presented in Figure 6 can also be implemented on a test device with multiple fluid flow paths, which are present on one or more lateral flow membranes. For instance, in one embodiment, one "high affinity" capture zone is present in one fluid flow path, and the "low affinity" capture zone is present in a second fluid flow path. This design avoids the potential for depletion of the analyte at the first capture zone, which might skew the results at the downstream second capture zone. The fluid flow paths are optionally formed on a single substrate by an impermeable barrier; and/or may be present on separate lateral flow substrates.

The capture zone with the high affinity antibodies captures and retains a larger amount of analyte, and therefore shows a visible signal at a lower analyte concentration, than the capture zone with the low affinity capture antibodies. In this design, the number of capture zones can be readily extended to more than two, through the use of more than two antibody affinities. Antibodies can be deposited either directly or through a binding agent such as BSA or IgG or amino dextran or other protein molecule to which the antibody has been conjugated. Such binding agents can help amplify a signal because of increased density of antibody on the binding agent, or because the binding agent may have a stronger interaction for the matrix material, or because the linkage forms a "tether" allowing greater avidity or binding capacity of the antibody by the reduction in steric hindrance.

In other embodiments, at least one of the second immunoreagents is not immobilized at its capture zone, but is conjugated to a binding moiety that directs it to a particular capture zone. Such binding interactions act to "sort" high affinity and low affinity immunoreagents during sample flow, and such binding moieties include biotin/streptavidin or complementary oligonucleotides (or derivatives thereof).

For example, as illustrated in **Figure 7****,** the capture reagent immobilized in the low threshold capture zone is streptavidin, which captures a biotin-conjugated immunoreagent (e.g., the second binding member). A low affinity antibody may be directly immobilized at the high threshold capture zone. The streptavidin/biotin interaction illustrated in Figure 7 may optionally be substituted with another binding pair described herein, such as complementary oligonucleotides, including derivatives such as pRNA and others as described herein. These capture zones may further be oriented serially, on one fluid flow path, or on separate fluid flow paths.

The capture zones may employ a variety of specific binding interactions to capture or immobilize the detectable immunocomplexes containing analyte, and these interactions include antigen/antibody, biotin/avidin, carbohydrate/lectin, complementary oligonucleotides (including pyranosyl RNA or DNA, or other oligonucleotide derivatives), receptor/ligand molecules including hormone/hormone receptor, enzyme/cofactor, or specific protein interaction, and metal/chelator.

In certain embodiments, an oligonucleotide is bound directly or indirectly (e.g., through a chemical linker) to the surface of the lateral flow substrate at the capture zone(s), with the complementary oligonucleotide conjugated to an immunoreagent for capturing the immunocomplex (see **Figure 8**). Different capture sequences then provide the basis for discriminating different antibody reagents having varying analyte affinity.

In some embodiments, the oligonucleotide is selected from DNA, RNA, DNA-RNA hybrids, oligonucleotides comprising locked nucleic acids, pyranosyl-RNA (pRNA), pyranosyl-DNA (pDNA), 2'-O-methyl oligonucleotides, and 5'-5' inverted oligonucleotides. The use of oligonucleotide agents to capture oligonucleotide conjugates is described in U.S. Patent Application Publication 2005/0208576.

The complementary oligonucleotides may have the same or similar hybridization efficiencies, and conjugated or linked to high and low affinity antibodies, to thereby provide the basis for discriminating different threshold levels of analyte. See **Figure 8****.** Because the higher affinity antibody complexes to a greater extent with the analyte, the zone where the higher affinity antibody has bound will capture and retain a larger amount of analyte, and therefore show a visible signal at a lower sample analyte concentration, than the zone where the lower affinity antibody is captured.

Alternatively, the concentration of the same specific binding pair member can be varied (e.g., immobilized) in each capture zone to allow for the capture of different levels of a target analyte. The zones with a relatively higher concentrations of immobilization reagent thus capture more analyte and produce a positive signal at lower analyte concentrations. For example, streptavidin may be applied to the capture zones at concentrations of from 0.1 mg/mL to about 10 mg/mL, or from 0.1 to 5 mg/mL, thereby forming different capture zones with different detectable thresholds of a biotin conjugated and labeled immunocomplex formed in the presence of analyte.

In one embodiment, a test device includes a single lateral flow membrane with a single fluid flow path, which comprises serial capture zones (*see, e.g.,* **Figures 7** and **8**). In this embodiment, in the event of high affinity capture binding in the first capture zone, especially when the analyte is present at low concentration in the test sample, it is possible that a significant portion of the analyte in the sample would be captured on the first capture zone. For example, in a typical capture zone on a lateral flow immunoassay, a capture zone 1 mm x 5 mm can retain about 1 µg, or approximately 6 picomoles, of capture IgG (Brown (2009). Antibodies: Key to a Robust Lateral Flow Immunoassay. In Lateral Flow Immunoassay, edited by Wong and Tse, Humana Press, New York, p. 60). A 100 µL sample which includes cTnI at a concentration 0.04 ng/mL (1.5 x 10⁻¹⁶ moles of analyte), could therefore be entirely depleted by one capture zone since the capture molecule is present at over 10⁴ -fold excess.

Similarly, for NT-proBNP, multiple cutoffs on a single test device would be useful since the clinical decision points are about 125 pg/mL and about 450 pg/mL (which are recommended CHF decision threshold values for individuals under 75 years of age and 75 years and older, respectively). These concentrations correspond to 1.5 x 10⁻¹⁵ and 5.4 x 10⁻¹⁵ mole of analyte, respectively, in a 100 µL sample. The analyte in a sample would be affected by a capture zone that retains on the order of 10⁻¹² moles of analyte molecules. Therefore, for this analyte, serial capture zones that do not account for depletion of analyte on a lateral flow device would not be practical.

In certain embodiments, the device architecture exploits the analyte depletion phenomenon. A lateral flow test device, in one embodiment, is formed by layering multiple capture zones as sequential lines or regions, or by layering a capture zone as a continuous area formed of deposited capture moieties, on the test membrane. As analyte and reagent react and migrate along the device and are bound to the capture molecules, the analyte is removed from the migrating solution. Then, the number of detectable capture regions or length of the capture region is directly related to the quantity of analyte molecules originally present in the sample, and these embodiments are thereby able to extend the quantitative range of the device. The device according to this embodiment acts like a gauge to detect analyte levels, and is particularly useful for expanding the range of the assay.

In the case of a continuous capture area, the portion of the capture zone having visible label will be directly related to the quantity of analyte originally present in the sample. In this manner the amount of analyte could be measured in a semi-quantitative, thermometer-like fashion *(see* **Figure 1**).

### Labels

At least one immunoreagent or binding member has a detectable moiety (e.g., a label). Exemplary labels include particulate labels such as colloidal metallic particles such as gold, colloidal non-metallic particles such as selenium or tellurium, dyed or colored particles such as a dyed plastic, dyed impregnated latex particles, organic polymer latex particles and liposomes, nanospheres, colored beads, polymer microcapsules, or other vesicles containing directly visible substances, and the like.

As for assays that do not directly detect label (e.g., with the naked eye), a fluorescent label such as fluorescein, europium, cyanine, or alexa dyes, a chemiluminescent label, a fluorescent-energy transfer label, a fluorescent quantum dot, a paramagnetic label, an electrochemical label using amperometric (either with direct or oscillating applied voltage) or carbon nanotube detection methods, can be used.

For example, detectable molecules include but are not limited to fluorophores as well as others known in the art as described, for example, in Principles of Fluorescence Spectroscopy, Joseph R. Lakowicz (Editor), Plenum Pub Corp, 2nd edition (July 1999) and the 6th Edition of the Molecular Probes Handbook by Richard P. Hoagland.

Directly visible labels include incorporation of dye-impregnated latex or other particles which have more functional groups than gold particles for conjugating to antibody, or are more visible than gold. In certain examples, silver-coated gold particles are employed, which can enhance the signal by one or two orders of magnitude. The use of quantum dots or nanospheres as colorimetric labels might also enhance signal. The use of larger gold particles (e.g., greater than the standard 40 nm diameter particle) may also provide enhanced sensitivity.

The label-bearing specific binding pair members may be diffusively bound to the lateral flow membrane upstream of the capture/detection zone. In these embodiments, when the sample migrates along a fluid flow path on the lateral flow membrane, it encounters the labeled specific binding pair member(s), and the analyte, if present in the sample, binds the label-bearing specific binding pair member. The analyte-specific binding pair member-label complex further migrates to the capture zone, and binds the capture binding pair member. Excess label can then be washed away.

The present invention provides a device, as described above, for the detection of multiple levels of one analyte and, disclosed herein, is the detection of multiple analytes, or multiple levels of multiple analytes. In order to detect the presence of the test analyte, the analyte must be labeled, so that it can be visualized. Visualization can occur with or without the use of an external reader instrument.

In certain examples, the device uses an external reader to provide an objective result. The reader may provide a displayed, printable, or downloadable result in various examples. Use of an external reader may be advantageous such that labels of greater sensitivity may be employed, such as fluorescent labels.

The reader further provides the option of communication capabilities (e.g., via internet, cable, wireless, or GSM etc.) to automatically notify a healthcare or emergency healthcare professional upon a particular result requiring emergency action, or may be connected to an institution's health records, to store records of tests. The reader further provides various memory options, to acquire (for example, through alphanumeric input or through automatic barcode reading) the operator ID and patient ID, as well as date and time of test results, for detailed patient histories.

### EXAMPLES

The present invention is further illustrated by reference to the following illustrative Examples.

### Example 1 - Evaluation of a two-level test device for detecting the presence of cardiac troponin I

In a lateral flow test device with four serial capture zones, sample comprising 10 ng/mL cTnI was applied. Over half the analyte was depleted by the first line (*see* **Figure 9** and Table 1, below).

| **Table 1.** | | |
|---|---|---|
| Capture zone no.* | Average Signal, RFU | % total analyte captures |
| 1 | 57467 | 74.1 |
| 2 | 16621 | 21.4 |
| 3 | 2178 | 2.8 |
| 4 | 1246 | 1.6 |
| * (number 1 indicates closest to sample application zone | | |

### Example 2 - Evaluation of a two-level test device for detecting the presence of cardiac troponin I

The purpose of the following study was to demonstrate the ability of an exemplary device to distinguish two concentrations of cardiac troponin I (cTnI) in a qualitative/semi-quantitative manner.

### Reagents

Mouse monoclonal anti-cTnI antibody attached to colored microspheres, ∼0.02%.

Rabbit polyclonal anti-cTnI antibody conjugated to biotin, ~10 µg/mL.

Lateral flow membrane with immobilized streptavidin-carrier complex (SA). The low-sensitivity strips were prepared by applying SA to the membrane at ∼0.5 mg/mL and the high-sensitivity strips were prepared with SA at ∼2 mg/mL.

### Samples tested

The samples tested by the assay were (1) Li-heparin human plasma, pooled, normal (NHP), and (2) NHP with recombinant cTnI diluted in it at fixed concentrations (concentrations confirmed by commercial cTnI immunoassay).

### Test Device and Assay

The test device comprises one high sensitivity lateral flow membrane ("strip") and one low-sensitivity lateral flow membrane. The membranes are positioned parallel to one another, similar to the device shown in **Figure 3****.** Two lateral flow membranes, one low-sensitivity and one high-sensitivity, are paired in a single device parallel to each other; sample application section of the device allows sample to flow freely towards both membranes. The test device does not include an impermeable barrier, as there is a space between the lateral flow membranes.

Three sets of samples were tested, each in triplicate - (1) blank NHP (*i.e.,* no recombinant cTnI), (2) NHP with recombinant cTnI at ∼50 pg/mL and (3) NHP with recombinant cTnI at ∼500 pg/mL.

Samples were applied at ∼240 µL per test device, incubated for approximately 15 minutes at ambient conditions. Each test device was then read visually and on a portable reflectometer at ∼550nm (e.g., 520 to 550 nm).

### Results

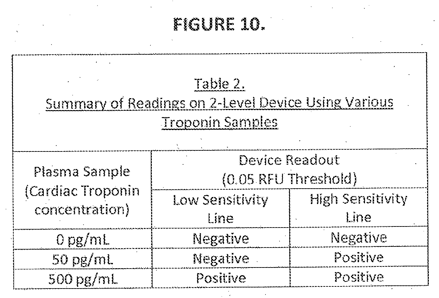

Example of the collected data is given in the table 2 and **Figure 10****.** These results demonstrate that two-level test device design allows detection of analyte at low concentration, *e.g.,* ∼50 pg cTnI/mL on high-sensitivity strips only, and detection of analyte at high concentrations, e.g. ∼500 pg/mL, on both strips.

Analysis of the signal distributions within three standard deviation (SD) range of its average value on low-sensitivity strips indicated absence of analyte detection at low concentration.

| **Table 2.** | | | | | | |
|---|---|---|---|---|---|---|
| **Sample** | **Normal Human Plasma (NHP)** | | **recombinant cTnI at 50 pg/mL in NHP** | | **recombinant cTnI at 500 pg/mL in NHP** | |
| strip sensitivity | low | high | low | high | low | high |
| rep 1 signal | 0.0267 | 0.0165 | 0.0163 | 0.0733 | 0.0872 | 0.269 |
| rep 2 signal | 0.0142 | 0.0169 | 0.0217 | 0.0884 | 0.0973 | 0.315 |
| rep 3 signal | 0.0200 | 0.0182 | 0.0117 | 0.0896 | 0.1060 | 0.381 |
| average signal | 0.0203 | 0.0172 | 0.0166 | 0.0838 | 0.0968 | 0.3217 |
| SD | 0.0063 | 0.0009 | 0.0050 | 0.0091 | 0.0094 | 0.0563 |
| average - 3 SD | 0.00153 | 0.01453 | 0.00155 | 0.05651 | 0.06861 | 0.15278 |
| average + 3 SD | 0.03907 | 0.01987 | 0.03158 | 0.11102 | 0.12506 | 0.49056 |
| visual read | negative | negative | negative | positive | positive | positive |

## Claims

1. A device for the detection of at least two threshold levels of a marker of myocardial injury in a patient sample comprising:
one sample application zone;
one or more fluid flow paths formed by one or more lateral flow substrates, and in fluid communication with the one sample application zones; wherein the one or more fluid flow paths are formed by one or more bibulous, absorbent, and/or porous membranes, wherein at least one of the one or more fluid flow paths further comprises membranes or porous pads of various porosity to filter cells and/or debris, which are located in proximity to the one sample application zone, wherein the one or more fluid flow paths comprise a first marker capture zone and a second marker capture zone, wherein the first and second capture zones are in series on a single flow path or are in separate flow paths, and wherein the first capture zone is configured to detectably capture the marker when present in the sample at or above a first threshold level, and the second capture zone is configured to detectably capture the marker when present in the sample at or above a second threshold;
a first binding member having an attached or bound detectable label capable of forming a capturable and detectable complex with the marker in the capture zones; and
a second binding member capable of forming a capturable and detectable complex with the marker in the capture zones; wherein the second binding member is either immobilized as a continuous area formed by deposited second binding member along the capture zones or is capable of being immobilized during sample flow as a continuous area formed by deposited second binding member along the capture zones,
the marker being detectable in the sample by formation of a capturable and detectable complex formed with the first binding member and the second binding member that are included in the device;
wherein the marker is a cardiac troponin,
wherein the at least two threshold levels of marker are determined by the length of detectable complex binding along the two marker capture zones on each of the one or more fluid flow paths, thereby extending the range of detection,
wherein the marker is detected at a first threshold level in the patient sample and a second threshold level in the patient sample,
wherein the first threshold level corresponds to 0.04 ng/ml and is indicative of presence of the marker in blood circulation, and
wherein the second threshold level corresponds to 0.1 ng/ml of marker and is indicative of an acute cardiac event.

2. The device of claim 1, wherein the cardiac troponin is cTnI.

3. The device of claim 1 or claim 2, further comprising a control zone in each flow path as in indication of sample flow.

4. The device of claim 3, wherein the second binding member includes a moiety capturable by said capture zones.

5. The device of claim 4, wherein the second binding member is captured by a streptavidin-biotin interaction, an antibody-antigen interaction, or by hybridization of complementary or substantially complementary oligonucleotides.

6. The device of any one of claims 1 to 5, wherein the first and second binding members are antibodies or antigen-binding portions thereof.

7. The device of any one of claims 1 to 6, wherein the first capture zone and the second capture zone are in parallel or bidirectional flow paths.

8. The device of claim 7, wherein the first capture zone and the second capture zone are in multiple radially arranged flow paths around a single sample application zone.

9. The device of claim 7 or claim 8, comprising three or more flow paths, each configured for detecting a different marker threshold level.

10. The device of one of claims 1 to 9, wherein the first and second binding members are the same capture agent at different concentrations in each capture zone.

11. The device of any one of claims 1 to 10, wherein the first marker capture zone_comprises an antibody with a first affinity for cTnI and the second marker capture zone comprises an antibody with a second affinity for cTnI.

12. A semi-quantitative method for evaluating the level of a marker of myocardial injury in a sample, the method comprising:
applying a test sample to the test device of any of claims 1 to 11; and
detecting the presence or absence of a detectable signal in the continuous capture zone(s); and
determining a plurality of threshold levels of the marker, wherein the plurality of thresholds comprises a first threshold level and a second threshold level of the marker.

13. The method of claim 12, wherein a detectable signal indicating the first threshold level of the marker is suggestive of a first decision, and a detectable signal indicating said second threshold level is suggestive of a second decision.

14. The method of claim 12 or 13, wherein the patient has one or more symptoms of a myocardial infarction.

15. The method of claim 13 or 14, wherein first threshold level of cTnI suggests that the patient condition be monitored for signs and symptoms of a myocardial infarction over a one to ten hour period.

16. The method of claim 15, wherein the second threshold level of cTnI suggests that the patient by treated for potential myocardial infraction.

17. The method of claim 15, wherein the method is repeated within one to ten hours to detect rising levels of cTnI.

18. The method of any one of claims 12 to 17, wherein the patient has a chronic heart condition, or other condition associated with a detectable level of circulating cTnI.

19. The method of any one of claims 12 to 17, wherein the test is performed at home or at point of care.

## Patentansprüche

1. Vorrichtung zum Erfassen von wenigstens zwei Schwellenwerten eines Markers einer myokardialen Verletzung in einer Patientenprobe, umfassend:
eine Probenauftragszone;
einen oder mehrere Fluidströmungswege, die durch ein oder mehrere laterale Strömungssubstrate gebildet sind und mit der einen Probenauftragszone in Fluidverbindung stehen;
wobei der eine oder die mehreren Fluidströmungswege durch eine oder mehrere saugfähige, absorbierende und/oder poröse Membran(en) gebildet sind, wobei mindestens einer des einen oder der mehreren Fluidströmungswege ferner Membranen oder poröse Kissen mit unterschiedlicher Porosität umfasst, um Zellen und/oder Fremdkörper zu filtern, die sich in der Nähe der einen Probenauftragszone befinden, wobei der eine oder die mehreren Fluidströmungsweg(e) eine erste Markererfassungszone und eine zweite Markererfassungszone umfassen, wobei die erste und zweite Erfassungszone in Serie in einem einzelnen Strömungsweg oder in separaten Strömungswegen angeordnet sind, und wobei die erste Erfassungszone dazu konfiguriert ist, den Marker nachweisbar zu erfassen, wenn er in der Probe bei oder über einem ersten Schwellenwert vorhanden ist, und die zweite Erfassungszone dazu konfiguriert ist, den Marker nachweisbar zu erfassen, wenn er in der Probe bei oder über einem zweiten Schwellwert vorhanden ist;
ein erstes Bindungselement mit einer angebrachten oder angebundenen nachweisbaren Kennzeichnung, das geeignet ist, mit dem Marker in den Erfassungszonen einen erfassbaren und nachweisbaren Komplex zu bilden; und
ein zweites Bindungselement, das geeignet ist, mit dem Marker in den Erfassungszonen einen erfassbaren und nachweisbaren Komplex zu bilden;
wobei das zweite Bindungselement entweder als eine durchgehende Fläche immobilisiert ist, die durch abgelagerte zweite Bindungselemente entlang der Erfassungszonen gebildet wird, oder dazu geeignet ist, während der Probenströmung als durchgehende Fläche immobilisiert zu werden, die durch abgelagerte zweite Bindungselemente entlang der Erfassungszonen gebildet wird, wobei der Marker in der Probe durch Bildung eines erfassbaren und nachweisbaren Komplexes nachweisbar ist, der mit dem ersten Bindungselement und dem zweiten Bindungselement gebildet wird, die in der Vorrichtung enthalten sind;
wobei der Marker ein kardiales Troponin ist, wobei die mindestens zwei Schwellenwerte des Markers durch die Länge der nachweisbaren Komplexbindung entlang der zwei Marker-Erfassungszonen auf jedem des einen oder der mehreren Fluidströmungspfade(s) bestimmt werden, wodurch der Nachweisbereich erweitert wird, wobei der Marker bei einem ersten Schwellenwert in der Patientenprobe und einem zweiten Schwellenwert in der Patientenprobe nachgewiesen wird, wobei der erste Schwellenwert 0,04 ng/ml entspricht und das Vorhandensein des Markers im Blutkreislauf anzeigt, und wobei der zweite Schwellenwert 0,1 ng/ml Marker entspricht und ein akutes Herzereignis anzeigt.

2. Vorrichtung nach Anspruch 1, wobei das kardiale Troponin cTnl ist.

3. Vorrichtung nach Anspruch 1 oder Anspruch 2, die ferner eine Kontrollzone in jedem Strömungsweg als Anzeige der Probenströmung umfasst.

4. Vorrichtung nach Anspruch 3, wobei das zweite Bindungselement eine Komponente enthält, die durch die Erfassungszonen erfassbar sind.

5. Vorrichtung nach Anspruch 4, wobei das zweite Bindungselement durch eine Streptavidin-Biotin-Wechselwirkung, eine Antikörper-Antigen-Wechselwirkung oder durch Hybridisieren von komplementären oder im wesentlichen komplementären Oligonukleotiden erfasst wird.

6. Vorrichtung nach einem der Ansprüche 1 bis 5, wobei die ersten und zweiten Bindungselemente Antikörper oder Antigen-bindende Abschnitte davon sind.

7. Vorrichtung nach einem der Ansprüche 1 bis 6, wobei sich die erste Erfassungszone und die zweite Erfassungszone in parallelen oder bidirektionalen Strömungspfaden befinden.

8. Vorrichtung nach Anspruch 7, wobei sich die erste Erfassungszone und die zweite Erfassungszone in mehreren radial angeordneten Strömungspfaden um eine einzelne Probenauftragszone befinden.

9. Vorrichtung nach Anspruch 7 oder Anspruch 8, die drei oder mehr Strömungswege umfasst, die jeweils dazu konfiguriert sind, einen unterschiedlichen Marker-Schwellenwert zu erfassen.

10. Vorrichtung nach einem der Ansprüche 1 bis 9, wobei das erste und das zweite Bindungselement bei verschiedenen Konzentrationen in jeder Erfassungszone das gleiche Erfassungsmittel sind.

11. Vorrichtung nach einem der Ansprüche 1 bis 10, wobei die erste Marker-Erfassungszone einen Antikörper mit einer ersten Affinität für cTnl und die zweite Marker-Erfassungszone einen Antikörper mit einer zweiten Affinität für cTnl umfasst.

12. Semiquantitatives Verfahren zum Bewerten des Grades einer myokardialen Verletzung in einer Probe, wobei das Verfahren umfasst:
Aufbringen einer Testprobe auf die Testvorrichtung nach einem der Ansprüche 1 bis 11; und
Nachweisen des Vorhandenseins oder Nichtvorhandenseins eines nachweisbaren Signals in der (den) durchgehenden Erfassungszone(n); und
Bestimmen mehrerer Schwellenwerte des Markers, wobei die mehreren Schwellenwerte einen ersten Schwellenwert und einen zweiten Schwellenwert des Markers umfassen.

13. Verfahren nach Anspruch 12, wobei ein nachweisbares Signal, das den ersten Schwellenwert des Markers anzeigt, eine erste Entscheidung nahelegt, und ein nachweisbares Signal, das den zweiten Schwellwert anzeigt, eine zweite Entscheidung nahelegt.

14. Verfahren nach Anspruch 12 oder 13, wobei der Patient ein oder mehrere Symptome eines Myokardinfarkts aufweist.

15. Verfahren nach Anspruch 13 oder 14, wobei der erste Schwellenwert von cTnl nahelegt, dass der Zustand des Patienten während einer Dauer von einer bis zu zehn Stunden auf Anzeichen und Symptome eines Myokardinfarkts überwacht werde.

16. Verfahren nach Anspruch 15, wobei der zweite Schwellenwert cTnl nahelegt, dass der Patient auf einen potentiellen Myokardinfarkt hin behandelt werde.

17. Verfahren nach Anspruch 15, wobei das Verfahren innerhalb von einer bis zehn Stunden wiederholt wird, um steigende cTnI-Spiegel nachzuweisen.

18. Verfahren nach einem der Ansprüche 12 bis 17, wobei der Patient eine chronische Herzerkrankung oder eine andere Erkrankung in Verbindung mit einem nachweisbaren Spiegel an zirkulierendem cTnl aufweist.

19. Verfahren nach einem der Ansprüche 12 bis 17, wobei der Test zu Hause oder an einer medizinischen Versorgungsstelle durchgeführt wird.

## Revendications

1. Dispositif permettant la détection d'au moins deux niveaux de seuil d'un marqueur de lésion du myocarde chez un échantillon de patient comprenant :
une zone d'application d'un échantillon ;
une ou plusieurs voies d'écoulement de fluide formées par un ou plusieurs substrats à écoulement latéral et en communication fluidique avec les zones d'application d'un échantillon ;
la ou les voies d'écoulement de fluide sont formées par une ou plusieurs membranes spongieuses, absorbantes et/ou poreuses, au moins l'une des voies d'écoulement de fluide comprenant en outre des membranes ou des tampons poreux à porosité diverse de manière à filtrer les cellules et/ou ou les débris qui sont situés à proximité de la zone d'application d'un échantillon, la ou les voies d'écoulement de fluide comprenant une première zone de capture de marqueur et une seconde zone de capture de marqueur, les première et seconde zones de capture sont en série sur une seule voie d'écoulement ou sont dans des voies d'écoulement séparées, et la première zone de capture étant conçue pour capturer de manière détectable le marqueur lorsqu'il est présent dans l'échantillon au niveau ou au-dessus d'un premier niveau de seuil, et la seconde zone de capture étant conçue pour capturer de manière détectable le marqueur lorsqu'il est présent dans l'échantillon au niveau ou au-dessus d'un second niveau de seuil ;
un premier élément de liaison comportant une étiquette détectable attachée ou liée capable de former un complexe pouvant être capturé et détecté avec le marqueur dans les zones de capture ; et
un second élément de liaison capable de former un complexe pouvant être capturé et détecté avec le marqueur dans les zones de capture ;
le deuxième élément de liaison étant soit immobilisé en tant que zone continue formée par le deuxième élément de liaison déposé le long des zones de capture, soit pouvant être immobilisé pendant le flux d'échantillon en tant que zone continue formée par le second élément de liaison déposé le long des zones de capture, le marqueur étant détectable dans l'échantillon par la formation d'un complexe capturable et détectable formé avec le premier élément de liaison et le deuxième élément de liaison qui sont inclus dans le dispositif ;
le marqueur étant une troponine cardiaque, les au moins deux niveaux de seuil de marqueur étant déterminés par la longueur de la liaison du complexe détectable le long des deux zones de capture du marqueur sur chacune des voies d'écoulement de fluide, élargissant ainsi la plage de détection, le marqueur étant détecté à un premier niveau de seuil dans l'échantillon de patient et à un second niveau de seuil dans l'échantillon de patient, le premier niveau de seuil correspondant à 0,04 ng/ml et indiquant la présence du marqueur dans la circulation sanguine, et le second niveau de seuil correspondant à 0,1 ng/ml de marqueur et indiquant un événement cardiaque aigu.

2. Dispositif selon la revendication 1, dans lequel la troponine cardiaque est cTnl.

3. Dispositif selon la revendication 1 ou 2, comprenant en outre une zone de contrôle dans chaque voie d'écoulement, comme indication du flux d'échantillon.

4. Dispositif selon la revendication 3, dans lequel le second élément de liaison comprend une fraction pouvant être capturée par lesdites zones de capture.

5. Dispositif selon la revendication 4, dans lequel le second élément de liaison est capturé par une interaction streptavidine-biotine, par une interaction anticorps-antigène ou par hybridation d'oligonucléotides complémentaires ou essentiellement complémentaires.

6. Dispositif selon l'une quelconque des revendications 1 à 5, dans lequel les premier et second éléments de liaison sont des anticorps ou des parties de liaison à l'antigène s'y rapportant.

7. Dispositif selon l'une quelconque des revendications 1 à 6, dans lequel la première zone de capture et la seconde zone de capture sont dans des voies d'écoulement parallèles ou bidirectionnelles.

8. Dispositif selon la revendication 7, dans lequel la première zone de capture et la seconde zone de capture se situent dans plusieurs voies d'écoulement disposées radialement autour d'une zone d'application à un seul échantillon.

9. Dispositif selon la revendication 7 ou la revendication 8, comprenant trois voies de circulation ou plus, chacune étant conçue de manière à détecter un niveau de seuil de marqueur différent.

10. Dispositif selon l'une des revendications 1 à 9, dans lequel les premier et second éléments de liaison sont le même agent de capture à des concentrations différentes dans chaque zone de capture.

11. Dispositif selon l'une quelconque des revendications 1 à 10, dans lequel la première zone de capture de marqueur comprend un anticorps ayant une première affinité pour cTnl et la seconde zone de capture de marqueur comprend un anticorps ayant une seconde affinité pour cTnl.

12. Procédé semi-quantitatif permettant l'évaluation du niveau d'un marqueur de lésion du myocarde dans un échantillon, le procédé comprenant :
l'application d'un échantillon d'essai sur le dispositif de test selon l'une quelconque des revendications 1 à 11 ; et
la détection de la présence ou de l'absence d'un signal détectable dans la ou les zones de capture continue ; et
la détermination d'une pluralité de niveaux de seuil du marqueur, la pluralité de seuils comprenant un premier niveau de seuil et un second niveau de seuil du marqueur.

13. Procédé selon la revendication 12, dans lequel un signal détectable indiquant le premier niveau de seuil du marqueur suggère une première décision, et un signal détectable indiquant ledit second niveau de seuil suggère une seconde décision.

14. Procédé selon la revendication 12 ou 13, dans lequel le patient présente un ou plusieurs symptômes d'infarctus du myocarde.

15. Procédé selon la revendication 13 ou 14, dans lequel le premier seuil de cTnl suggère que l'état du patient soit surveillé afin de déceler les signes et symptômes d'infarctus d'un myocarde sur une période comprise entre une et dix heures.

16. Procédé selon la revendication 15, dans lequel le second niveau de seuil de cTnl suggère que le patient soit traité pour un éventuel infarctus du myocarde.

17. Procédé selon la revendication 15, dans lequel le procédé est répété dans un délai compris entre une et dix heures de manière à détecter les taux croissants de cTnl.

18. Procédé selon l'une quelconque des revendications 12 à 17, dans lequel le test est effectué à domicile ou au point d'intervention.

19. Procédé selon l'une quelconque des revendications 12 à 17, dans lequel le test est effectué à domicile ou au point d'intervention.
